(19) Europäisches Patentamt
European Patent Office
Office européen des brevets

(11) Veröffentlichungsnummer: **0 057 864**
**B1**

(12) # EUROPÄISCHE PATENTSCHRIFT

(45) Veröffentlichungstag der Patentschrift: **03.10.84**

(51) Int. Cl.³: **C 07 D 405/06**, A 01 N 43/50, A 01 N 43/64, A 01 N 43/60

(21) Anmeldenummer: **82100583.2**

(22) Anmeldetag: **28.01.82**

(54) **2-Azolylmethyl-1,3-dioxolan- und -dioxan-Derivate, Verfahren zu ihrer Herstellung und ihre Verwendung als Fungizide.**

(30) Priorität: **07.02.81 DE 3104311**

(43) Veröffentlichungstag der Anmeldung: **18.08.82 Patentblatt 82/33**

(45) Bekanntmachung des Hinweises auf die Patenterteilung: **03.10.84 Patentblatt 84/40**

(84) Benannte Vertragsstaaten: **AT BE CH DE FR GB IT LI NL SE**

(56) Entgegenhaltungen:
**EP - A - 0 060 962**
**DE - A - 2 803 870**
**DE - A - 2 804 096**
**DE - A - 2 943 631**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Krämer, Wolfgang, Dr., Am Eckbusch 39/45, D-5600 Wuppertal 1 (DE)**
Erfinder: **Büchel, Karl Heinz, Prof.Dr., Dabringhausenerstrasse 42, D-5093 Burscheid (DE)**
Erfinder: **Elbe, Hans-Ludwig, Dr., Dasnoeckel 59, D-5600 Wuppertal 11 (DE)**
Erfinder: **Kraatz, Udo, Dr., Koernerstrasse 6, D-5090 Leverkusen 1 (DE)**
Erfinder: **Reiser, Wolf, Dr., Kiebitzweg 12a, D-5600 Wuppertal 1 (DE)**
Erfinder: **Schulze, Andreas, Dr., Voiswinkeler Strasse 35, D-5060 Bergisch-Gladbach 2 (DE)**
Erfinder: **Regel, Erik, Ing.grad, Bergerheide 72a, D-5600 Wuppertal 1 (DE)**
Erfinder: **Brandes, Wilhelm, Dr., Eichendorffstrasse 3, D-5653 Leichlingen (DE)**
Erfinder: **Frohberger, Paul-Ernst, Dr., Willi-Baumeister-Strasse 5, D-5090 Leverkusen (DE)**

**EP 0 057 864 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 2-Azolylmethyl-1,3-dioxolan- und dioxan-Derivate, mehrere Verfahren zu ihrer Herstellung sowie ihre Verwendung als Fungizide.

Es ist bereits bekannt geworden, dass N-Halogenalkylmercapto-imide, wie beispielsweise N-Trichlormethylthiotetrahydrophthalimid, gute fungizide Eigenschaften aufweisen [vergleiche R. Wegler, Chemie der Pflanzenschutz- und Schädlingsbekämpfungsmittel, Band 2, S. 108 (1970)]. Ausserdem ist bereits bekannt, dass Triazolylethyl-benzylether, wie beispielsweise [1-(2,4-Dichlorphenyl)-2-(1,2,4-triazol-1-yl)-ethyl]-(2,6-dichlorbenzyl)- oder -(3,4-dichlorbenzyl)-ether, gute fungizide Wirkung besitzen (vergleiche DE-OS 2 547 953). Weiterhin sind schon von N-Ethylazonen 1,3-Dioxolanyl-Derivate bekannt (vergleiche DE-OS 2 804 096).

Die Wirkung all dieser Verbindungen ist jedoch in bestimmten Indikationsbereichen, insbesondere bei niedrigen Aufwandmengen und -konzentrationen, nicht immer ganz befriedigend.

Es wurden neue 2-Azolylmethyl-1,3-dioxolan- und -dioxan-Derivate der allgemeinen Formel

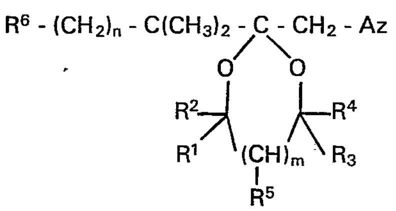 (I)

in welcher

Az für Imidazol-1-yl oder 1,2,4-Triazol-1-yl steht,

$R^1$ für Wasserstoff oder Alkyl mit 1 bis 4 Kohlenstoff-Atomen steht,

$R^2$ für Wasserstoff oder Alkyl mit 1 bis 4 Kohlenstoff-Atomen steht,

$R^3$ für Wasserstoff oder Alkyl mit 1 bis 4 Kohlenstoff-Atomen steht,

$R^4$ für Wasserstoff oder geradkettiges und verzweigtes Alkyl mit 1 bis 4 Kohlenstoffatomen steht, das gegebenenfalls substituiert sein kann durch: Hydroxy, Alkoxy mit 1 bis 4 Kohlenstoffatomen, Dialkylamino, Dialkylaminocarbonyl und Alkylcarbonyloxy mit jeweils 1 bis 2 Kohlenstoffatomen in jedem Alkylteil, gegebenenfalls substituiertes Phenoxy und gegebenenfalls substituiertes Phenylalkoxy mit 1 bis 4 Kohlenstoffatomen im Alkylteil, gegebenenfalls substituiertes Phenylcarbonyloxy und gegebenenfalls substituiertes Phenylalkylcarbonyloxy mit 1 bis 4 Kohlenstoffatomen im Alkylteil, Alkylsulfonyloxy mit 1 bis 4 Kohlenstoffatomen und gegebenenfalls substituiertes Phenylsulfonyloxy, wobei jeweils als Phenyl- oder Phenoxysubstituenten genannt seien: Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, wie insbesondere Fluor- und Chloratomen, Dimethylamino, Acetylamino, Acetyl-methylamino, gegebenenfalls durch Methyl oder Acetyl substituiertes Piperazinyl; sowie schliesslich für gegebenenfalls substituiertes Phenyl und Phenylalkyl mit 1 bis 4 Kohlenstoffatomen im Alkylteil steht, wobei als Phenylsubstituenten die oben genannten Phenylsubstituenten in Frage kommen;

$R^1$ und $R^3$ ausserdem gemeinsam für eine gegebenenfalls durch Alkyl mit 1 bis 4 Kohlenstoffatomen substituierte Tetra- oder Pentamethylenbrücke stehen;

$R^5$ für Wasserstoff oder Alkyl mit 1 bis 4 Kohlenstoff-Atomen steht;

m für die Zahlen 0 oder 1 steht;

$R^6$ für Wasserstoff, Halogen, Cyano, geradkettiges oder verzweigtes Alkyl mit 1 bis 4 Kohlenstoff-Atomen und für gegebenenfalls substituiertes Phenyl steht, wobei als Phenylsubstituenten die bei $R^4$ bereits genannten Phenylsubstituenten in Frage kommen, sowie für die Gruppierungen $-X-R^7$, $-COOR^8$ und $-CONHR^9$ steht, wobei

X für Sauerstoff, Schwefel, die SO- oder $SO_2$-Gruppe steht;

$R^7$ für geradkettiges oder verzweigtes Alkyl mit 1 bis 4 Kohlenstoff-Atomen, Halogenalkyl mit 1 bis 2 Kohlenstoff- und 1 bis 5 gleichen oder verschiedenen Halogen-Atomen, für Cyano, sowie für gegebenenfalls substituiertes Phenyl und Phenylalkyl mit 1 bis 4 Kohlenstoff-Atomen im Alkylteil steht, wobei jeweils als Phenylsubstituenten die bei $R^4$ bereits genannten Phenylsubstituenten in Frage kommen;

$R^8$ für geradkettiges oder verzweigtes Alkyl mit 1 bis 4 Kohlenstoff-Atomen steht;

$R^9$ für geradkettiges oder verzweigtes Alkyl mit 1 bis 4 Kohlenstoff-Atomen sowie für gegebenenfalls substituiertes Phenyl steht, wobei als Phenylsubstituenten die bei $R^4$ bereits genannten Phenylsubstituenten in Frage kommen, und

n für die Zahlen 0 oder 1 steht,

jedoch mit der Massgabe, dass der Rest $R^6$-$(CH_2)_n$- nicht für Methyl stehen darf, wenn m für die Zahl 0, Az für den 1,2,4-Triazol-1-yl-Rest und $R^4$ für gegebenenfalls substituiertes Phenoxyalkyl steht,

sowie deren pflanzenverträglichen Säureadditions-Salze und Metallsalz-Komplexe gefunden.

Die Verbindungen der Formel (I) können gegebenenfalls in verschiedenen Stereoisomeren vorkommen; vorzugsweise fallen sie in Form von Stereoisomerengemischen an.

Weiterhin wurde gefunden, dass man die 2-Azolylmethyl-1,3-dioxolan- und -dioxan-Derivate der Formel (I) erhält, wenn man

a) substituierte 1,3-Dioxolan- und Dioxan-Derivate der Formel

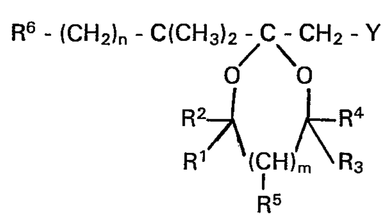 (II)

in welcher
$R^1$ bis $R^6$, m und n die oben angegebene Bedeutung haben und
Y für Halogen, insbesondere Chlor oder Brom, sowie die Gruppierung $-O-SO_2-Z$ steht, wobei
Z für Methyl oder p-Methylphenyl steht,
mit Alkylisalzen von Azolen der Formel

$$M - Az \qquad (III)$$

in welcher
Az die oben angegebene Bedeutung hat und
M für ein Alkalimetall steht,
in Gegenwart eines Verdünnungsmittels umsetzt, oder
b) Azolylmethyl-keto-Derivate der Formel

$$R^6 - (CH_2)_n - C(CH_3)_2 - \underset{\substack{\| \\ O}}{C} - CH_2 - Az \qquad (IV)$$

in welcher
Az, $R^6$ und n die oben angegebene Bedeutung haben, mit Diolen der Formel

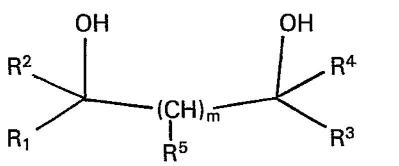

(V)

in welcher
$R^1$ bis $R^5$ und m die oben angegebene Bedeutung haben,
in Gegenwart eines Verdünnungsmittels und in Gegenwart einer Säure als Katalysator umsetzt.

An die so erhaltenen Verbindungen der Formel (I) können gegebenenfalls anschliessend eine Säure oder ein Metallsalz addiert werden. In manchen Fällen erweist es sich als vorteilhaft, die Verbindungen der Formel (I) über ihre Salze in reiner Form zu erhalten.

Die neuen 2-Azolylmethyl-1,3-dioxolan- und dioxan-Derivate der Formel (I) weisen starke fungizide Eigenschaften auf. Dabei zeigen überraschenderweise die erfindungsgemässen Verbindungen eine bessere fungizide Wirkung als die aus dem Stand der Technik bekannten Verbindungen N-Trichlormethylthio-tetrahydrophthalimid und [1-(2,4-Dichlorphenyl)-2-(1,2,4-triazol-1-yl)-ethyl]-(2,6-dichlorbenzyl)- bzw. -(3,4-dichlorbenzyl)-ether, welche wirkungsmässig ähnliche Verbindungen sind. Die erfindungsgemässen Stoffe stellen somit eine Bereicherung der Technik dar.

Die erfindungsgemässen 2-Azolylmethyl-1,3-dioxolan- und -dioxan-Derivate sind durch die Formel (I) allgemein definiert.

Bevorzugt sind diejenigen Verbindungen der Formel (I), in denen
$R^1$, $R^2$ und $R^3$ die in der bevorzugten Erfindungsdefinition angegebene Bedeutung haben;
$R^4$ für Wasserstoff oder geradkettiges und verzweigtes Alkyl mit 1 bis 4 Kohlenstoffatomen steht, das gegebenenfalls substituiert sein kann durch: Hydroxy, Methoxy, Ethoxy, Dimethylamino, Dimethylaminocarbonyl, gegebenenfalls substituiertes Phenoxy und Benzyloxy, Methylcarbonyloxy, Ethylcarbonyloxy, gegebenenfalls substituiertes Phenylcarbonyloxy und Benzylcarbonyloxy, Methylsulfonyloxy, Ethylsulfonyloxy sowie gegebenenfalls substituiertes Phenylsulfonyloxy, wobei jeweils als Phenylsubstituentengenannt seien: Fluor, Chlor, Methyl, Ethyl, Methoxy, Methylthio, Trifluormethyl und Trifluormethylthio, Dimethylamino, Acetylamino, Acetyl-methylamino, und 4-Acetylpiperazin-1--yl, sowie auch für gegebenenfalls substituiertes Phenyl und Benzyl steht, sowie als Phenylsubstituenten die obengenannten in Frage kommen;
$R^1$ und $R^3$ ausserdem gemeinsam für eine Tetramethylenbrücke stehen;
$R^5$ für Wassertoff, Methyl oder Ethyl steht;
$R^6$ für Wasserstoff, Fluor, Chlor, Brom, Methyl, Ethyl, Propyl, gegebenenfalls substituiertes Phenyl, wobei als Substituenten die bei $R^4$ bereits genannten Phenylsubstituenten in Frage kommen, sowie für die Gruppierungen $-X-R^7$, $-COOR^8$ und $-CONHR^9$ steht,
$R^7$ für Methyl, Ethyl, Trifluormethyl, Cyano, sowie gegebenenfalls substituiertes Phenyl und Benzyl steht, wobei jeweils als Phenylsubstituenten die bei $R^4$ bereits genannten Phenylsubstituenten in Frage kommen;
$R^8$ für Methyl oder Ethyl steht;
$R^9$ für Methyl, Ethyl sowie gegebenenfalls substituiertes Phenyl steht, wobei als Phenylsubstituenten die bei $R^4$ bereits genannten Phenylsubstituenten in Frage kommen;
n für 1 steht; und
Az, X und m die in der Erfindungsdefinition angegebene Bedeutung haben.

Verwendet man beispielsweise 2-Brommethyl-2--[β-(4-Chlorphenoxy)-α,α-dimethyl]-ethyl-4-ethyl--1,3-dioxolan und Imidazol-natrium als Ausgangsstoffe, so kann der Reaktionsablauf durch das folgende Formelschema wiedergegeben werden (Verfahren a):

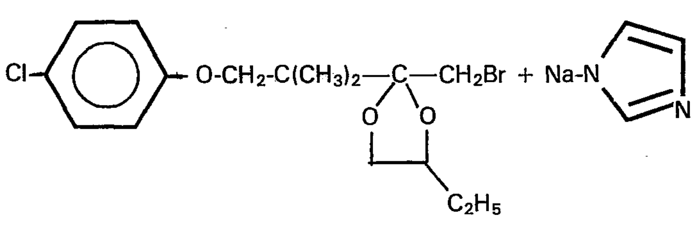

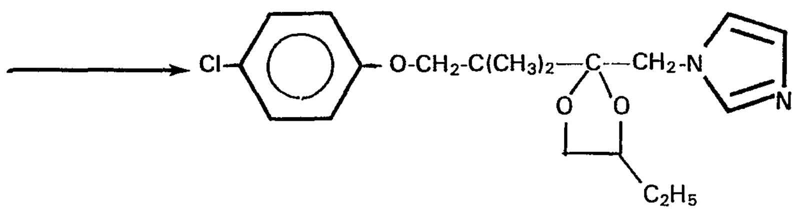

Verwendet man beispielsweise 4-(4-Chlorphenoxy)-3,3-dimethyl-1-(1,2,4-triazol-1-yl)-butan-2-on und 1,2-Butandiol als Ausgangsstoffe, so kann der Reaktionsablauf durch das folgende Formelschema wiedergegeben werden (Verfahren b):

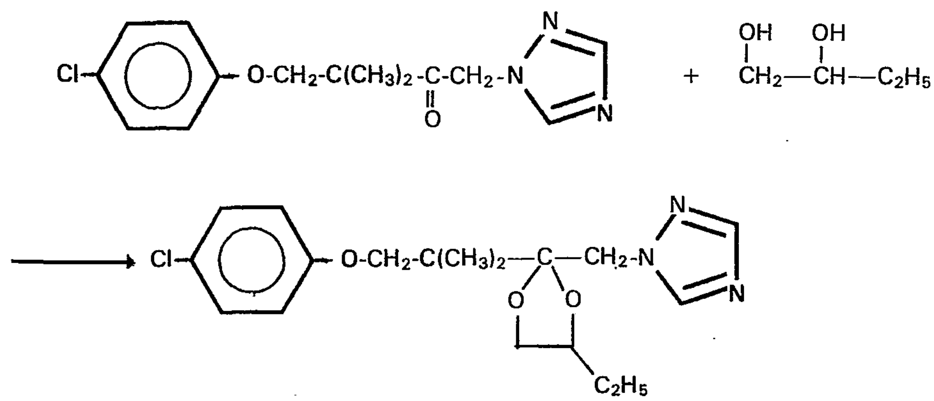

Die bei der Durchführung des erfindungsgemässen Verfahrens (a) als Ausgangsstoffe benötigten 2-Halogenmethyl-1,3-dioxolan- und -dioxan-Derivate sind durch die Formel (II) allgemein definiert. In dieser Formel stehen $R^1$, $R^2$, $R^3$, $R^4$, $R^5$, $R^6$ und die Indizes m und n vorzugsweise für die Bedeutungen, die bei der Beschreibung der erfindungsgemässen Stoffe der Formel (I) bereits vorzugsweise genannt wurden.

Die substituierten 1,3-Dioxolan- und Dioxan-Derivate der Formel (II) sind noch nicht bekannt. Sie werden erhalten, indem man Keto-Derivate der Formel

$$R^6 - (CH_2)_n - C(CH_3)_2 - \underset{\underset{O}{\|}}{C} - CH_2 - Y \qquad \text{(VI)}$$

in welcher

Y, $R^6$ und n die oben angegebene Bedeutung haben, mit Diolen der Formel (V) gemäss den Bedingungen des Verfahrens (b) umsetzt.

Die Keto-Derivate der Formel (V) sind bekannt (vergleiche (DE-OS 2 635 663 und die veröffentlichten Europäischen Patentanmeldungen 0 042 980 und 0 054 865). Sie werden z.B. erhalten, indem man die entsprechenden Ketone mit Chlor oder Brom in Gegenwart eines inerten organischen Lösungsmittels, wie beispielsweise Ether, chlorierten oder nicht chlorierten Kohlenwasserstoffen, bei Raumtemperatur umsetzt, oder mit üblichen Chlorierungemitteln, wie beispielsweise Sulfurylchlorid, bei 20 bis 60°C umsetzt.

Die ausserdem für das erfindungsgemässe Verfahren (a) als Ausgangsstoffe zu verwendenden Alkalisalze von Azolen sind durch die Formel (III) allgemein definiert. In dieser Formel hat Az vorzugsweise die in der allgemeinen Erfindungsdefinition angegebene Bedeutung. M steht vorzugsweise für Natrium und Kalium.

Die Alkalisalze von Azolen der Formel (III) sind allgemein bekannt. Sie werden durch Umsetzung von Imidazol bzw. 1,2,4-Triazol mit Natrium- oder Kaliummethylat oder durch Umsetzung von Imidazol bzw. Triazol mit der äquivalenten Menge des entsprechenden Alkalihydrids erhalten.

Die für die Durchführung des erfindungsgemässen Verfahrens (b) als Ausgangsstoffe zu verwendenden Azolylmethyl-keto-Derivate sind durch die Formel (IV) allgemein definiert. In dieser Formel stehen Az, $R^6$ und der Index n vorzugsweise für die Bedeutungen, die bei der Beschreibung der erfindungsgemässen Stoffe der Formel (I) bereits vorzugsweise genannt wurden.

Die Azolylmethyl-keto-Derivate der Formel (IV) sind bekannt (vergleiche z.B. DE-OS 2 431 407 und DE-OS 2 906 061, sowie die veröffentlichten Europäischen Patetanmeldungen 0 044 993 und 0 054 865). Sie werden erhalten indem man Keto-Derivate der Formel (VI) mit Alkalisalzen von Azolen der Formel (III) gemäss den Bedingungen des Verfahrens (a) umsetzt, oder mit Azolen direkt in üblicher Weise in Gegenwart eines Säurebinders umsetzt.

Die ausserdem für das erfindungsgemässe Verfahren (b) als Ausgangsstoffe zu verwendenden Diole sind durch die Formel (V) allgemein definiert. In dieser Formel stehen $R^1$, $R^2$, $R^3$, $R^4$, $R^5$ und der Index m vorzugsweise für die Bedeutungen, die bei der Beschreibung der erfindungsgemässen Stoffe der Formel (I) bereits vorzugsweise genannt wurden.

Die Diole der Formel (V) sind allgemein bekannte Verbindungen der organischen Chemie bzw. werden sie in allgemein bekannter Art und Weise erhalten.

Als Verdünnungsmittel kommen für das erfindungsgemässe Verfahren (a) inerte organische Lösungsmittel in Frage. Hierzu gehören vorzugsweise Amide, wie Dimethylformamid oder Dimethylacetamid; ferner Dimethylsulfoxid oder Hexamethylphosphorsäuretriamid.

Die Reaktionstemperaturen können bei der Durchführung des erfindungsgemässen Verfahrens (a) in einem grösseren Bereich variiert werden. Im allgemeinen arbeitet man zwischen 20 und 150°C, vorzugsweise bei 60 bis 150°C.

Bei der Durchführung des erfindungsgemässen Verfahrens (a) setzt man auf 1 Mol der Verbindungen der Formel (II) vorzugsweise 1 bis 2 Mol des Azol-Alkalisalzes der Formel (III) ein. Die Isolierung der Verbindung der Formel (I) erfolgt in üblicher Art und Weise.

Als Verdünnungsmittel kommen für das erfindungsgemässe Verfahren (b) inerte organische Lösungsmittel in Frage. Hierzu gehören aromatische Kohlenwasserstoffe, wie Benzol, Toluol oder Xylol, sowie halogenierte aliphatische und aromatische Kohlenwasserstoffe, wie Tetrachlorkohlenstoff, Chloroform, Methylenchlorid, Chlorbenzol oder Dichlorbenzol sowie Gemische dieser Lösungsmittel mit Alkoholen wie z.B, Butanol. Es kann aber auch gegebenenfalls ein entsprechender Überschuss an Diol der Formel (V) verwendet werden.

Das erfindungsgemässe Verfahren (b) wird in Gegenwart einer starken Säure als Katalysator durchgeführt. Hierzu gehören vorzugsweise die Chlorwasserstoffsäure, Bromwasserstoffsäure, Schwefelsäure sowie insbesondere die p-Toluolsulfonsäure.

Die Reaktionstemperaturen können bei der Durchführung des erfindungsgemässen Verfahrens (b) in einem grösseren Bereich variiert werden. Im allgemeinen arbeitet man zwischen 40 und 180°C, vorzugsweise bei 80 b is 180°C. Gegebenenfalls kann auch unter erhöhtem Druck gearbeitet werden.

Bei der Durchführung des erfindungsgemässen Verfahrens (b) setzt man auf 1 Mol der Verbindung der Formel (IV) vorzugsweise 1 bis 2 Mol Diol der Formel (V) sowie katalytische Mengen Säure ein. Die Isolierung der Verbindungen der Formel (I) erfolgt in üblicher Art und Weise.

Zur Herstellung von physiologisch verträglichen Säureadditionssalzen der Verbindungen der Formel (I) kommen vorzugsweise folgende Säuren in Frage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis VI. Haupt- und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, Isolieren und gegebenenfalls durch Umkristallisation reinigen.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe mit besonders gutem Erfolg zur Bekämpfung von Venturia-Arten, wie gegen der Erreger des Apfelschorfs (Fusicladium dendriticum), von Uromyces- und Puccinia-Arten, wie gegen den Erreger des Bohnenrostes (Uromyces phaseoli) und des Braunrostes am Weizen (Puccinia recondita), sowie zur Bekämpfung von Getreidekrankheiten, wie gegen den Erreger des echten Getreidemehltaus (Erysiphe graminis) und gegen die Streifenkrankheit der Gerste (Helminthosporium gramineum) verwendet werden. Die erfindungsgemässen Verbindungen zeigen auch eine gute in-vitro-Wirkung, insbesondere gegen Krankheitserreger an Reispflanzen.

Hervorzuheben ist die teilweise systemische Wirkung der erfindungsgemässen Stoffe. So gelingt es, Pflanzen gegen Pilzbefall zu schützen, wenn man den Wirkstoff über den Boden und die Wurzel den oberirdischen Teilen der Pflanze zuführt.

In entsprechenden Aufwandmengen zeigen die erfindungsgemässen Stoffe auch wachstumsregulierende Eigenschaften.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Durck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösunsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-Azol- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

*Herstellungsbeispiele*

*Beispiel 1*

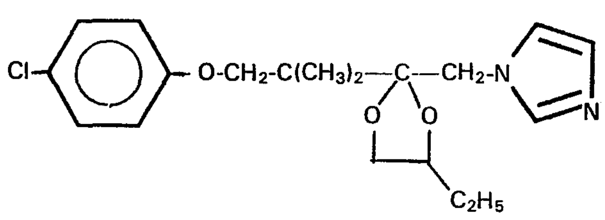

× HCl

Verrfahren (a)

25,8 g ($3,8 \times 10^{-1}$ Mol) imidazol werden in 600 ml Dimethylformamid gelöst, 20,5 g ($3,8 \times 10^{-1}$ Mol) Natriummethylat, gelöst in 60 ml Methanol, zugetropft und das Methanol abdestilliert. Bei 80°C werden 74 g ($1,9 \times 10^{-1}$ Mol) rohes 2-Brommethyl-2-[β-(4-chlorphenoxy)-α,α-dimethyl]-ethyl-1,3-dioxolan (Gehalt an Reinprodukte 62%) zugetropft und weitere 6 Stunden unter Rückfluss erhitzt. Nach Abkühlen wird 2 ℓ Wasser eingerührt, mit zweimal 500 ml Toluol extrahiert, die vereinigten Toluolphasen dreimal mit je 250 ml Wasser extrahiert und das Lösungsmittel im Wasserstrahlvakuum abdestilliert. Der Rückstand wird in 300 ml Diisopropylether aufgenommen und mit gesättigter etherischer Salzsäure versetzt. Der entstehende Niederschlag wird abgesaugt. Man erhält 39,7 g (84% der Theorie) 2-(Imidazol-1-yl)-methyl-2-[β-(4-chlorphenoxy)-α,α-dimethyl]-ethyl-4-ethyl-1,3-dioxolan-hydrochlorid vom Schmelzpunkt 146-47°C.

*Herstellung des Ausgangsproduktes*

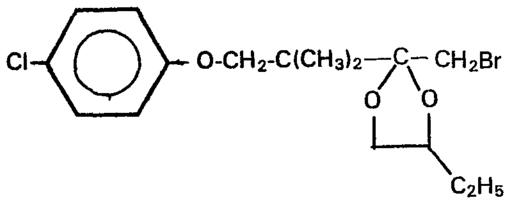

91 g (3 × $10^{-1}$ Mol) 1-Brom-3,3-dimethyl-4-(4-chlorphenoxy)-butan-2-on werden in 400 ml Toluol gelöst, 54 g (6 × $10^{-1}$ Mol) 1,2-Butandiol sowie 5,2 g (3 × $10^{-2}$ Mol) p-Toluolsulfonsäure zugegeben und das Reaktionsgemisch anschliessend 16 Stunden am Wasserabscheider unter Rückfluss erhitzt. Nach Abkühlen wird die organische Phase mit zweimal 250 ml gesättigter Natriumhydrogencarbonatlösung gewaschen und das Lösungsmittel im Wasserstrahlvakuum abdestilliert. Man erhält 120 g rohes 2-Brommethyl-2-[β-(4-chlorphenoxy)-α,α-dimethyl]-ethyl-4-ethyl-1,3-dioxoloan (gaschromatographisch ermittelter Gehalt an Reinprodukt 62%), das direkt gemäss Beispiel 1 weiter umgesetzt wird.

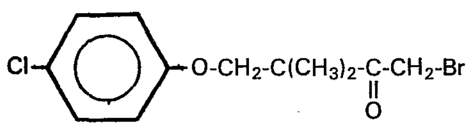

26 g (0,159 Mol) 1-(4-Chlorphenoxy)-2,2-dimethyl-butan-3-on werden in 300 ml Chloroform gelöst und bei 20°C tropfenweise so mit 25,5 g (0,159 Mol) Brom versetzt, dass laufend Entfärbung eintritt. Nach beendeter Zugabe lässt man 30 Minuten bei Raumtemperatur rühren und engt danach durch Abdestillieren des Lösungsmittels im Vakuum ein. Man erhält 48,5 (quantitativer Umsatz) 1-Brom-4-(4-chlorphenoxy)-3,3-dimethylbutan-2-on vom Siedepunkt 150-160°C/0,19 mbar.

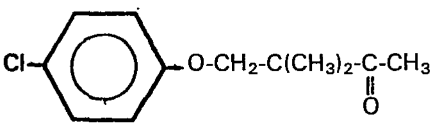

29,7 g (0,55 Mol) Natriummethylat werden in 500 ml Methanol gelöst und unter Rühren 70,4 g (0,55 Mol) 4-Chlorphenol versetzt. Nach 10 Minuten Rühren wird das Lösungsmittel unter vermindertem Druck abdestilliert und der Rückstand in 100 ml Glykol aufgenommen. Diese Lösung wird zu einer Lösung von 135 g (0,5 Mol) 2,2-Dimethyl-1-tosyloxy-butan-3-on in 200 ml Glykol gegeben. Man lässt 48 Stunden bei 100 bis 120°C rühren, kühlt ab und rührt das Reaktionsgemisch in 2000 ml Wasser ein man extrahiert zweimal mit je 250 ml Diethylether, wäscht die vereinigten organischen Phasen dreimal mit je 100 ml Wasser, einmal mit 100 ml 10%iger Natronlauge und nochmals mit 100 ml Wasser, trocknet über Natriumsulfat und destilliert.

Man erhält 62,9 g (55,7% der Theorie) 1-(4-Chlorphenoxy)-2,2-dimethyl-butan-3-on vom Siedepunkt 135-140°C/0,55 mbar.

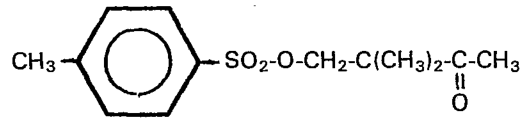

47,6 g (0,25 Mol) 4-Toluolsulfochlorid werden in 100 ml Chloroform gelöst, 35 g (0,3 Mol) 2,2-Dimethyl-1-hydroxy-butan-3-on zugegeben und bei 0 bis 5°C 40 ml (0,5 Mol) Pyridin zugetropft. Man lässt 15 Stunden bei Raumtemperatur nachrühren, gibt das Reaktionsgemisch auf 200 g Eis und 70 ml konzentrierte Salzsäure, trennt die organische Phase ab, wäscht sie dreimal mit je 200 ml Wasser nach, trocknet über Natriumsulfat und engt ein. Der Rückstand wird in 100 ml Petrolether aufgenommen, wobei das Endprodukt auskristallisiert. Man erhält 46 g (71% der Theorie) 2,2-Dimethyl-1-tosyloxy-butan-3-on als farblose Kristalle vom Schmelzpunkt 49-52°C.

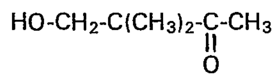

Zu 172 g (2 Mol) Methylisopropylketon in 1000 ml Methanol werden 66 g (2,2 Mol) Paraformaldehyd und 1 g Kaliumhydroxid in 10 ml Methanol getropft. Man erhitzt 15 Stunden unter Rückfluss und destilliert anschliessend das Methanol über eine Kolonne bei 82°C Innentemperatur ab. Der Rückstand wird im Wasserstrahlvakuum destilliert. Man erhält 152,7 g (68% der Theorie) 2,2-Dimethyl-1-hydroxy-butan-3-on vom Siedepunkt 80-82°C/16 mbar.

*Beispiel 2*

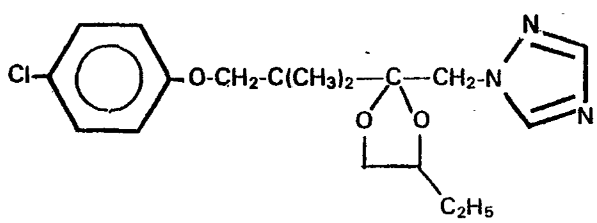

× HCl

(Verfahren a)

Zu 21,4 g (3,1 × $10^{-1}$ Mol) 1,2,4-Triazol in 600 ml Dimethylformamid werden 16,8 g (3,1 × $10^{-1}$ Mol) Natriummethylat in 60 ml Methanol getropft und das Methanol abdestilliert. Bei 80°C werden 60 g (1,56 × $10^{-1}$ Mol) 2-Brommethyl-2-[β-(4-chlorphenoxy)-α,α-dimethyl]-ethyl-4-ethyl-dioxolan (Gehalt an Reinprodukt 62%) zugetropft und das Reaktionsgemisch 15 Stunden unter Rückfluss erhitzt. Die abgekühlte Dimethylformamid-Lösung wird in 2 ℓ Wasser eingerührt und mit zweimal 250 ml Toluol extrahiert. Die Toluolphase wird mit dreimal 250 ml Wasser gewaschen, über Natriumsulfat getrocknet und das Lösungsmittel im Wasserstrahlva-

kuum abdestilliert. Man erhält 20 g rohes Produkt, das in 200 ml Diethylether aufgenommen und mit 20 ml gesättigter etherischer Salzsäure versetzt wird. Das Lösungsmittel wird abdestilliert und der Rückstand erneut in 200 ml Ether aufgenommen. Man erhält ein Öl, von dem die etherische Phase abdekantiert wird. Nach Chromatographiert an einer Kieselgel-Säule (250 g Kieselgel 60) in Chloroform-Methanol erhält man 7,8 g (17,4% der Theorie) 2-(1,2,4--Triazol-1-yl)-methyl-2-[β-(4-chlorphenoxy)-α,α-dimethyl]-ethyl-4-ethyl-1,3-dioxolan vom Schmelzpunkt 109°C.

In entsprechender Weise und gemäss den angegebenen Verfahren werden die folgenden Verbindungen der allgemeinen Formel

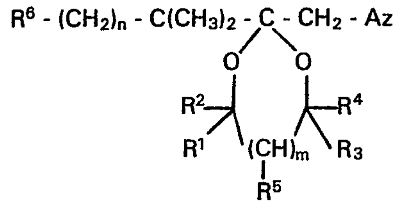 (I)

erhalten:

| Beispiel Nr. | 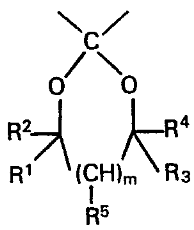 | $R^6$ | n | Az | Fp. (°C) |
|---|---|---|---|---|---|
| 3 | 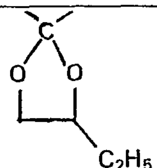 | H | 1 | 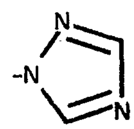 | 220 (× HCl) |
| 4 | 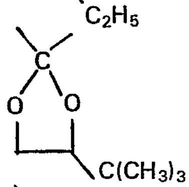 | H | 1 | » | 132 (× HCl) |
| 5 | 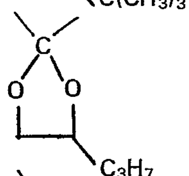 | H | 1 | » | 190-92 (× HCl) |
| 6 | 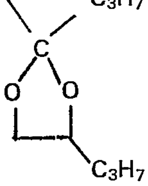 | 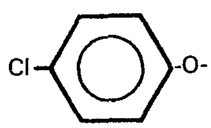 | 1 | » | 145 (× HCl) |
| 7 | 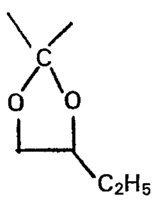 | 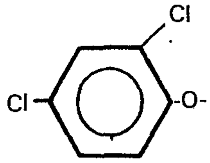 | 1 | » | 156 (× HCl) |
| 8 | 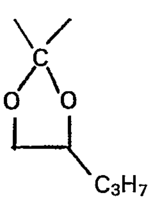 | 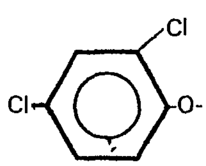 | 1 | » | 144 (× HCl) |

| Beispiel Nr. | 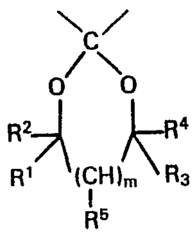 | $R^6$ | n | Az | Fp. (°C) |
|---|---|---|---|---|---|
| 9 | 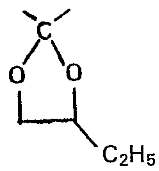 | 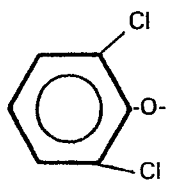 | 1 | 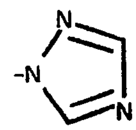 | 138 (× HCl) |
| 10 | 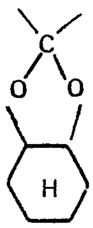 | 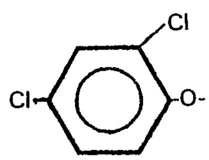 | 1 | » | 142 (× HCl) |
| 11 | 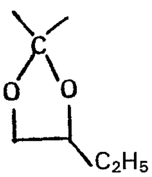 | 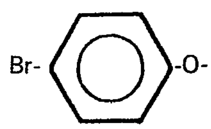 | 1 | » | 174 (× HCl) |
| 12 | 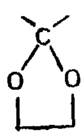 | 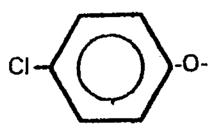 | 1 | 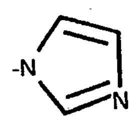 | 169 (× HCl) |
| 13 | 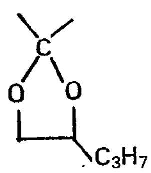 | 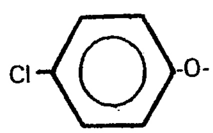 | 1 | » | zähes Öl |
| 14 | 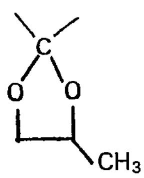 | 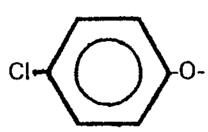 | 1 | » | 127 (× HCl) |
| 15 | 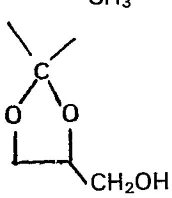 | 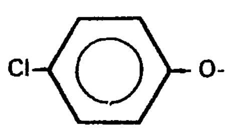 | 1 | » | 148 (× HCl) |

| Beispiel Nr. | 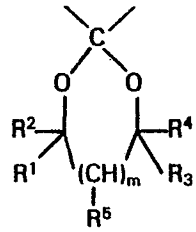 | $R^6$ | n | Az | Fp. (°C) |
|---|---|---|---|---|---|
| 16 | 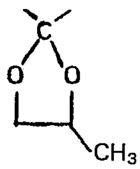 | 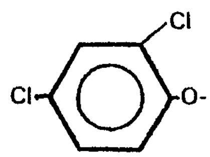 | 1 | 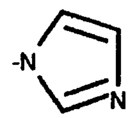 | 166 (× HCl) |
| 17 | 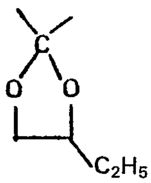 | 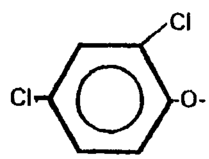 | 1 | » | 158 (× HCl) |
| 18 | 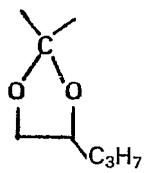 | 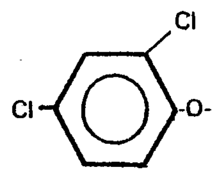 | 1 | » | 154 (× HCl) |
| 19 | 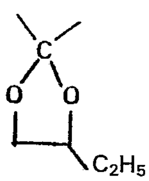 | 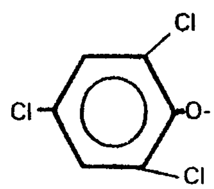 | 1 | » | 169 (× HCl) |
| 20 | 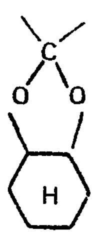 | 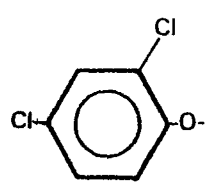 | 1 | » | 135 (× HCl) |
| 21 | 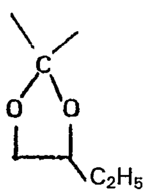 | 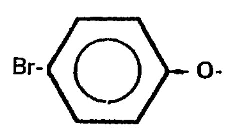 | 1 | » | 176 (× HCl) |
| 22 | 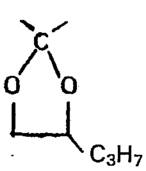 | H | 1 | » | 173 (× HCl) |

| Beispiel Nr. | 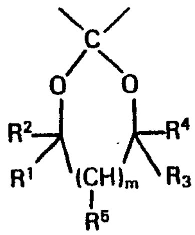 | $R^6$ | n | Az | Fp. (°C) |
|---|---|---|---|---|---|
| 23 | 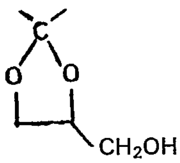 | H | 1 | 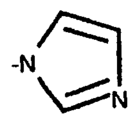 | 168 (× HCl) |
| 24 | 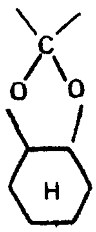 | H | 1 | » | 222 (× HCl) |
| 25 | 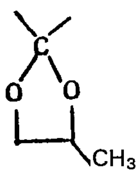 | 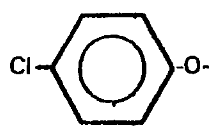 | 1 | » | zähes (Öl) |
| 26 | 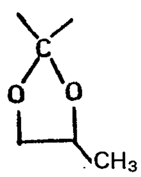 | 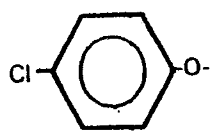 | 1 | » | 162 (× HCl) |
| 27 | 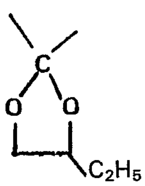 | 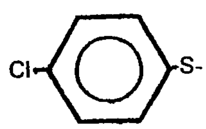 | 1 | » | 92 |
| 28 | 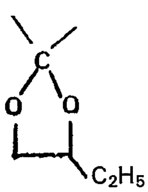 | 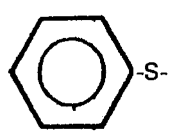 | 1 | » | zähes Öl |
| 29 | 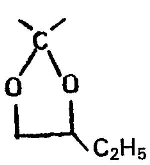 | 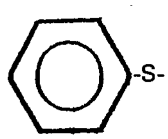 | 1 | 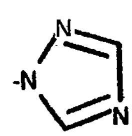 | zähes Öl |

| Beispiel Nr. | 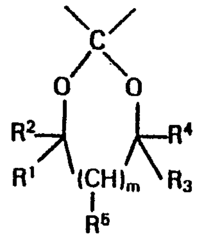 | $R^6$ | n | Az | Fp. (°C) |
|---|---|---|---|---|---|
| 30 | 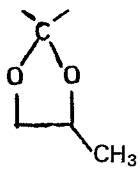 | 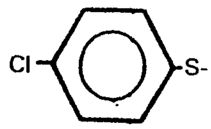 | 1 | 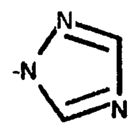 | zähes Öl |
| 31 | 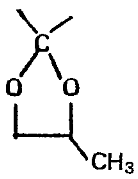 | 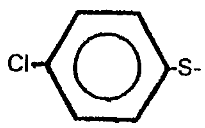 | 1 | 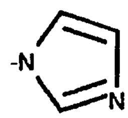 | zähes Öl |
| 32 | 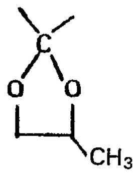 | 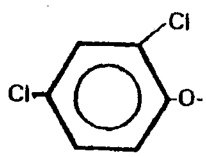 | 1 | 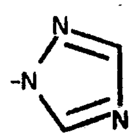 | 152 (× HCl) |
| 33 | 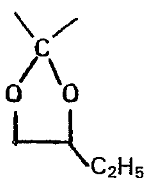 | 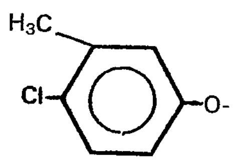 | 1 | 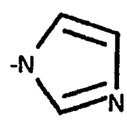 | 170 (× HCl) |
| 34 | 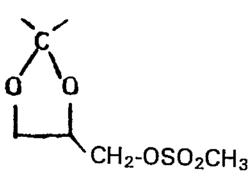 | 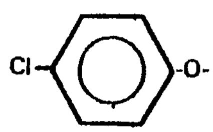 | 1 | 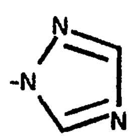 | Kirstall-brei |
| 35 | 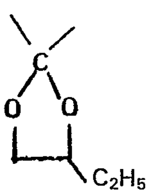 | 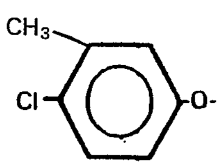 | 1 | 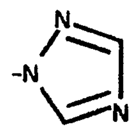 | 167 (× HCl) |
| 36 | 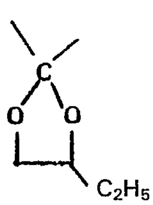 | 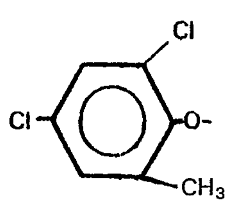 | 1 | 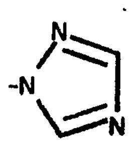 | 129 (× HCl) |

| Beispiel Nr. | 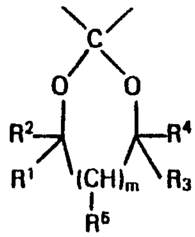 | $R^6$ | n | Az | Fp. (°C) |
|---|---|---|---|---|---|
| 37 | 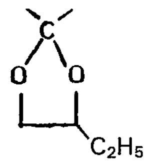 | 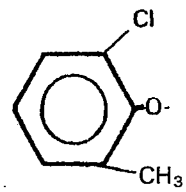 | 1 | 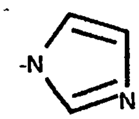 | 184 (× HCl) |
| 38 | 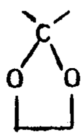 | 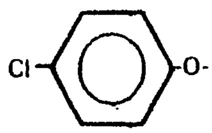 | 1 | 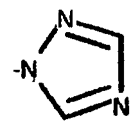 | 180-82 (× HCl) |
| 39 | 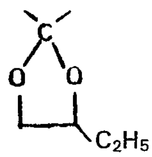 | 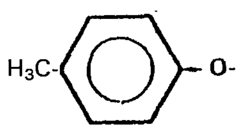 | 1 | 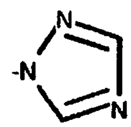 | $n_D^{20}$ = 1,5264 |
| 40 | 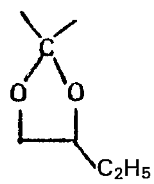 | 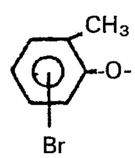 | 1 | 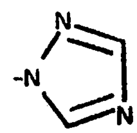 | $n_D^{20}$ = 1,5283 |
| 41 | 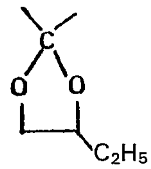 | 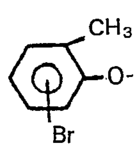 | 1 | 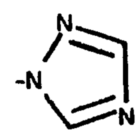 | 137 (× $\frac{1}{2}$ NDS)* |

NDS* = 1,5-Naphthalindisulfonsäure

*Anwendungsbeispiele*

In den nachfolgenden Beispielen werden die nachstehend angegebenen Verbindungen als Vergleichssubstanzen eingesetzt:

(A) 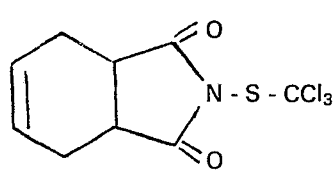

(B)

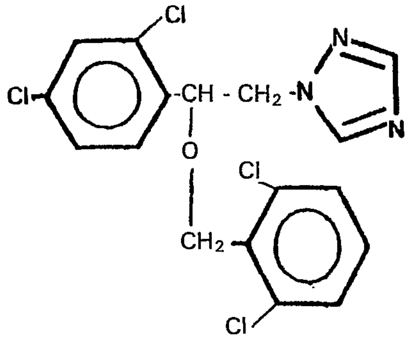

(C)

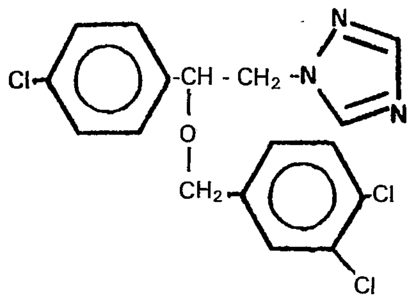

*Beispiel A*

Fusicladium-Test (Apfel) / protektiv

Lösungsmittel:
4,7 Gewichtsteile Aceton
Emulgator:
0,3 Gewichtsteile Alkylarylpolyglykolether
Wasser:
95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschliessend werden sie mit einer wässrigen Konidiensuspension des Apfelschorferregers (Fusicladium dendriticum) inokuliert und 18 Stunden lang in einer Feuchtkammer bei 18 bis 20°C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, dass die Pflanzen vollständig befallen sind.

Eine deutliche Überlegenheit in der Wirksamkeit gegenüber der aus dem Stand der Technik bekannten Verbindung (A) zeigen bei diesem Test z.B. die Verbindungen gemäss folgender Herstellungsbeispiele: 1, 2, 6, 7 und 8.

*Beispiel B*

Uromyces-Test (Bohnen) / protektiv

Lösungsmittel:
4,7 Gewichtsteile Aceton
Emulgator:
0,3 Gewichtsteile Alkylarylpolyglykolether
Wasser:
95,0 Gewichtsteile

Man vermischt die für die gewünschte Wirkstoffkonzentration in der Spritzflüssigkeit notwendige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man die jungen Bohnenpflanzen, die sich im 2-Blattstadium befinden, bis zur Tropfnässe. Die Pflanzen verbleiben zum Abtrocknen 24 Stunden bei 20-22°C und einer relativen Luftfeuchtigkeit von 70% im Gewächshaus. Anschliessend werden sie mit einer wässrigen Uredosporensuspension des Bohnenrosterregers (Uromyces phaseoli) inokuliert und 24 Stunden lang in einer dunklen Feuchtkammer bei 20-22°C und 100% relativer Luftfeuchtigkeit inkubiert.

Die Pflanzen werden dann unter intensiver Belichtung für 9 Tage bei 20-22°C und einer relativen Luftfeuchtigkeit von 70-80% im Gewächshaus aufgestellt.

10 Tage nach der Inokulation wird der Befall der Pflanzen bestimmt. Die erhaltenen Boniturwerte werden in Prozent Befall umgerechnet. 0% bedeutet keinen Befall, 100% bedeutet, dass die Pflanzen vollständig befallen sind.

Eine deutliche Überlegenheit in der Wirksamkeit gegenüber den aus dem Stand der Technik bekannten Verbindungen (B) und (C) zeigen bei diesem Test

z.B. die Verbindungen gemäss folgender Herstellungsbeispiele: 1, 2, 13, 25, 26, 16, 6, 7, 17, 18 und 8.

*Beispiel C*

Puccinia-Test (Weizen) / protektiv

Lösungsmittel:
100 Gewichtsteile Dimethylformamid
Emulgator:
0,25 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1%igen wässrigen Agarlösung inokuliert. Nach Antrocknen besprüht man die Pflanzen mit der Wirkstoffzubereitung taufeucht. Die Pflanzen verbleiben 24 Stunden bei 20°C und 100% relativer Luftfeuchtigkeit in einer Inkubationskabine.

Die Pflanzen werden im Gewächshaus bei einer Temperatur von ca. 20°C und einer relativen Luftfeuchtigkeit von ca. 80% aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäss folgender Herstellungsbeispiele: 13, 6, 7, 17, 18, 8 und 19.

*Beispiel D*

Gerstenmehltau-Test (Erysiphe graminis var.hordei) / systemisch (pilzliche Getreidesprosskrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 × 12 Korn in Blumentöpfe 2 cm tief in ein Gemisch aus einem Volumenteil Fruhstorfer Einheitserde und einem Volumenteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var.hordei bestäubt und bei 21-22°C und 80-90% rel.Luftfeuchte und 16stündiger Belichtung weiter kultiviert. Innerhalb von 6 Tagen bilden sich an den Blättern die typischen Mehltaupusteln aus.

Der Befallsgrad wird in Prozent des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Eine deutliche Überlegenheit in der Wirksamkeit gegenüber der aus dem Stand der Technik bekannten Verbindung (B) zeigen bei diesem Test z.B. die Verbindungen gemäss folgender Herstellungsbeispiele: 5 und 22.

*Beispiel E*

Saatgutbeizmittel-Test / Streifenkrankheit der Gerste (samenbürtige Mykose)

Zur Herstellung eines zweckmässigen Trockenbeizmittels verstreckt man den Wirkstoff mit einem Gemisch aus gleichen Gewichtsteilen Talkum und Kieselgur zu einer feinpulverigen Mischung mit der gewünschten Wirkstoffkonzentration.

Zur Beizung schüttelt man Gerstensaatgut, das durch Drechslera graminea (Syn. Helminthosporium gramineum) natürlich verseucht ist, mit dem Beizmittel in einer verschlossenen Glasflasche. Das Saatgut setzt man auf feuchten Filterscheiben in verschlossenen Petrischalen im Kühlschrank 10 Tage lang einer Temperatur von 4°C aus. Dabei wird die Keimung der Gerste und gegebenenfalls auch der Pilzsporen eingeleitet. Anschliessend sät man die vorgekeimte Gerste mit 2 × 50 Korn 3 cm tief in Frühstorfer Einheitserde und kultiviert sie im Gewächshaus bei Temperaturen um 18°C in Saatkästen, die täglich 16 Stunden dem Licht ausgesetzt werden. Innerhalb von 3 bis 4 Wochen bilden sich die typischen Symptome der Streiferkrankheit aus.

Nach dieser Zeit bestimmt man die Anzahl der kranken Pflanzen in Prozent der insgesamt aufgelaufenen Pflanzen. Der Wirkstoff ist um so wirksamer je weniger Pflanzen erkrankt sind.

Bei diesem Test zeigen z.B. die folgenden Verbindungen eine sehr gute Wirkung, die derjenigen der aus dem Stand der Technik bekannten Verbindung (B) überlegen ist:
Verbindungen gemäss Herstellungsbeispielen: 5, 1, 12 und 13.

**Patentansprüche**

1. 2-Azolylmethyl-1,3-dioxolan- und -dioxan-Derivate der allgemeinen Formel

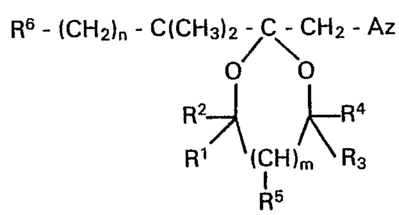

(I)

in welcher
Az für Imidazol-1-yl oder 1,2,4-Triazol-1-yl steht,
$R^1$ für Wasserstoff oder Alkyl mit 1 bis 4 Kohlenstoff-Atomen steht,
$R^2$ für Wasserstoff oder Alkyl mit 1 bis 4 Kohlenstoff-Atomen steht,

$R^3$ für Wasserstoff oder Alkyl mit 1 bis 4 Kohlenstoff-Atomen steht,

$R^4$ für Wasserstoff oder geradkettiges und verzweigtes Alkyl mit 1 bis 4 Kohlenstoffatomen steht, das gegebenenfalls substituiert sein kann durch: Hydroxy, Alkoxy mit 1 bis 4 Kohlenstoffatomen, Dialkylamino, Dialkylaminocarbonyl und Alkylcarbonyloxy mit jeweils 1 bis 2 Kohlenstoffatomen in jedem Alkylteil, gegebenenfalls substituiertes Phenoxy und gegebenenfalls substituiertes Phenylalkoxy mit 1 bis 4 Kohlenstoffatomen im Alkylteil, gegebenenfalls substituiertes Phenylcarbonyloxy und gegebenenfalls substituiertes Phenylalkylcarbonyloxy mit 1 bis 4 Kohlenstoffatomen im Alkylteil, Alkylsulfonyloxy mit 1 bis 4 Kohlenstoffatomen und gegebenenfalls substituiertes Phenylsulfonyloxy, wobei jeweils als Phenyl- oder Phenoxysubstituenten genannt seien: Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, wie insbesondere Fluor- und Chloratomen, Dimethylamino, Acetylamino, Acetyl-methylamino, gegebenenfalls durch Methyl oder Acetyl substituiertes Piperazinyl; sowie schliesslich für gegebenenfalls substituiertes Phenyl und Phenylalkyl mit 1 bis 4 Kohlenstoffatomen im Alkylteil steht, wobei als Phenylsubstituenten die oben genannten Phenylsubstituenten in Frage kommen;

$R^1$ und $R^3$ ausserdem gemeinsam für eine gegebenenfalls durch Alkyl mit 1 bis 4 Kohlenstoffatomen substituierte Tetra- oder Pentamethylenbrücke stehen;

$R^5$ für Wasserstoff oder Alkyl mit 1 bis 4 Kohlenstoff-Atomen steht;

m für die Zahlen 0 oder 1 steht;

$R^6$ für Wasserstoff, Halogen, Cyano, geradkettiges oder verzweigtes Alkyl mit 1 bis 4 Kohlenstoff-Atomen und für gegebenenfalls substituiertes Phenyl steht, wobei als Phenylsubstituenten die bei $R^4$ bereits genannten Phenylsubstituenten in Frage kommen, sowie für die Gruppierungen $-X-R^7$, $-COOR^8$ und $-CONHR^9$ steht, wobei

X für Sauerstoff, Schwefel, die SO- oder $SO_2$-Gruppe steht;

$R^7$ für geradkettiges oder verzweigtes Alkyl mit 1 bis 4 Kohlenstoff-Atomen, Halogenalkyl mit 1 bis 2 Kohlenstoff- und 1 bis 5 gleichen oder verschiedenen Halogen-Atomen, für Cyano, sowie für gegebenenfalls substituiertes Phenyl und Phenylalkyl mit 1 bis 4 Kohlenstoff-Atomen im Alkylteil steht, wobei jeweils als Phenylsubstituenten die bei $R^4$ bereits genannten Phenylsubstituenten in Frage kommen;

$R^8$ für geradkettiges oder verzweigtes Alkyl mit 1 bis 4 Kohlenstoff-Atomen steht;

$R^9$ für geradkettiges oder verzweigtes Alkyl mit 1 bis 4 Kohlenstoff-Atomen sowie für gegebenenfalls substituiertes Phenyl steht, wobei als Phenylsubstituenten die bei $R^4$ bereits genannten Phenylsubstituenten in Frage kommen, und

n für die Zahlen 0 oder 1 steht,

jedoch mit der Massgabe, dass der Rest $R^6-(CH_2)_n-$ nicht für Methyl stehen darf, wenn m für die Zahl 0, Az für den 1,2,4-Triazol-1-yl-Rest und $R^4$ für gegebenenfalls substituiertes Phenoxyalkyl steht, sowie deren pflanzenverträglichen Säureadditions-Salze und Metallsalz-Komplexe.

2. Verbindungen der allgemeinen Formel (I) in Anspruch 1, in welcher

$R^1$ für Wasserstoff oder geradkettiges und verzweigtes Alkyl mit 1 bis 4 Kohlenstoffatomen steht;

$R^2$ für Wasserstoff oder geradkettiges und verzweigtes Alkyl mit 1 bis 4 Kohlenstoffatomen steht;

$R^3$ für Wasserstoff oder geradkettiges und verzweigtes Alkyl mit 1 bis 4 Kohlenstoffatomen steht;

$R^4$ für Wasserstoff oder geradkettiges und verzweigtes Alkyl mit 1 bis 4 Kohlenstoffatomen steht, das gegebenenfalls substituiert sein kann durch: Hydroxy, Methoxy, Ethoxy, Dimethylamino, Dimethylaminocarbonyl, gegebenenfalls substituiertes Phenoxy und Benzyloxy, Methylcarbonyloxy, Ethylcarbonyloxy, gegebenenfalls substituiertes Phenylcarbonyloxy und Benzylcarbonyloxy, Methylsulfonyloxy, Ethylsulfonyloxy sowie gegebenenfalls substituiertes Phenylsulfonyloxy, wobei jeweils als Phenyl- oder Phenoxysubstituenten genannt seien: Fluor, Chlor, Methyl, Ethyl, Methoxy, Methylthio, Trifluormethyl, Trifluormethylthio, Dimethylamino, Acetylamino, Acetyl-methylamino und 4-Acetylpiperazin-1-yl, sowie auch für gegebenenfalls substituiertes Phenyl und benzyl steht, wobei als Phenylsubstituenten die obengenannten in Frage kommen;

$R^1$ und $R^3$ ausserdem gemeinsam für eine Tetramethylenbrücke stehen,

$R^5$ für Wasserstoff, Methyl oder Ethyl steht;

$R^6$ für Wasserstoff, Fluor, Chlor, Brom, Methyl, Ethyl, Propyl, gegebenenfalls substituiertes Phenyl, wobei als Substituenten die bei $R^4$ bereits genannten Phenylsubstituenten in Frage kommen, sowie für die Gruppierungen $-X-R^7$, $-COOR^8$ und $-CONHR^9$ steht, wobei

$R^7$ für Methyl, Ethyl, Trifluormethyl, Cyano, sowie gegebenenfalls substituiertes Phenyl und Benzyl steht, wobei jeweils als Phenylsubstituenten die bei $R^4$ bereits genannten Phenylsubstituenten in Frage kommen;

$R^8$ für Methyl oder Ethyl steht;

$R^9$ für Methyl, Ethyl sowie gegebenenfalls substituiertes Phenyl steht, wobei als Phenylsubstituenten die bei $R^4$ bereits genannten Phenylsubstituenten in Frage kommen, und

n für die Zahl 1 steht; und

Az, X und m die im Anspruch 1 angegebene Bedeutung haben;

jedoch mit der Massgabe, dass der Rest $R^6-(CH_2)_n-$ nicht für Methyl stehen darf, wenn m für die Zahl 0, Az für den 1,2,4-Triazol-1-yl-Rest und $R^4$ für gegebenenfalls substituiertes Phenoxyalkyl steht.

3. Verfahren zur Herstellung von 2-Azolymethyl--1,3-dioxolan- und -dioxan-Derivaten der allgemeinen Formel

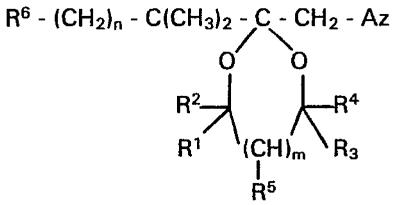
(I)

in welcher

Az für Imidazol-1-yl oder 1,2,4-Triazol-1-yl steht,

$R^1$ für Wasserstoff oder Alkyl mit 1 bis 4 Kohlenatoff-Atomen steht,

$R^2$ für Wasserstoff oder Alkyl mit 1 bis 4 Kohlenstoff-Atomen steht,

$R^3$ für Wasserstoff oder Alkyl mit 1 bis 4 Kohlenstoff-Atomen steht,

$R^4$ für Wasserstoff oder geradkettiges und verzweigtes Alkyl mit 1 bis 4 Kohlenstoffatomen steht, das gegebenenfalls substituiert sein kann durch: Hydroxy, Alkoxy mit 1 bis 4 Kohlenstoffatomen, Dialkylamino, Dialkylaminocarbonyl und Alkylcarbonyloxy mit jeweils 1 bis 2 Kohlenstoffatomen in jedem Alkylteil, gegebenenfalls substituiertes Phenoxy und gegebenenfalls substituiertes Phenylalkoxy mit 1 bis 4 Kohlenstoffatomen im Alkylteil, gegebenenfalls substituiertes Phenylcarbonyloxy und gegebenenfalls substituiertes Phenylalkylcarbonyloxy mit 1 bis 4 Kohlenstoffatomen im Alkylteil, Alkylsulfonyloxy mit 1 bis 4 Kohlenstoffatomen und gegebenenfalls substituiertes Phenylsulfonyloxy, wobei jeweils als Phenyl- oder Phenoxysubstituenten genannt seien: Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, wie insbesondere Fluor- und Chloratomen, Dimethylamino, Acetylamino, Acetyl-methylamino, gegebenenfalls durch Methyl oder Acetyl substituiertes Piperazinyl; sowie schliesslich für gegebenenfalls substituiertes Phenyl und Phenylalkyl mit 1 bis 4 Kohlenstoffatomen im Alkylteil steht, wobei als Phenylsubstituenten in Frage kommen;

$R^1$ und $R^3$ ausserdem gemeinsam für eine gegebenenfalls durch Alkyl mit 1 bis 4 Kohlenstoffatomen substituierte Tetra- oder Pentamethylenbrücke stehen;

$R^5$ für Wasserstoff oder Alkyl mit 1 bis 4 Kohlenstoff-Atomen steht;

m für die Zahlen 0 oder 1 steht;

$R^6$ für Wasserstoff, Halogen, Cyano, geradkettiges oder verzweigtes Alkyl mit 1 bis 4 Kohlenstoff-Atomen und für gegebenenfalls substituiertes Phenyl steht, wobei als Phenylsubstituenten die bei $R^4$ bereits genannten Phenylsubstituenten in Frage kommen, sowie für die Gruppierungen $-X-R^7$, $-COOR^8$ und $-CONHR^9$ steht, wobei

X für Sauerstoff, Schwefel, die SO- oder $SO_2$-Gruppe steht;

$R^7$ für geradkettiges oder verzweigtes Alkyl mit 1 bis 4 Kohlenstoff-Atomen, Halogenalkyl mit 1 bis 2 Kohlenstoff- und 1 bis 5 gleichen oder verschiedenen Halogen-Atomen, für Cyano, sowie für gegebenenfalls substituiertes Phenyl und Phenylalkyl mit 1 bis 4 Kohlenstoff-Atomen im Alkylteil steht, wobei jeweils als Phenylsubstituenten die bei $R^4$ bereits genannten Phenylsubstituenten in Frage kommen;

$R^8$ für geradkettiges oder verzweigtes Alkyl mit 1 bis 4 Kohlenstoff-Atomen steht;

$R^9$ für geradkettiges oder verzweigtes Alkyl mit 1 bis 4 Kohlenstoff-Atomen sowie für gegebenenfalls substituiertes Phenyl steht, wobei als Phenylsubstituenten die bei $R^4$ bereits genannten Phenylsubstituenten in Frage kommen, und

n für die Zahlen 0 oder 1 steht,

jedoch mit der Massgabe, dass der Rest $R^6-(CH_2)_n-$ nicht für Methyl stehen darf, wenn m für die Zahl 0, Az für den 1,2,4-Triazol-1-yl-Rest und $R^4$ für gegebenenfalls substituiertes Phenoxyalkyl steht,

sowie deren pflanzenverträglichen Säureadditions-Salze und Metallsalz-Komplexe,

dadurch gekennzeichnet, dass man

a) substituierte 1,3-Dioxolan- und Dioxan-Derivate der Formel

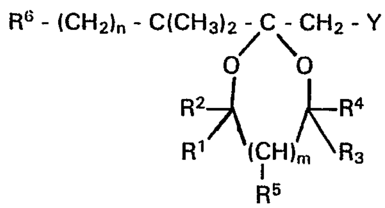
(II)

in welcher

$R^1$ bis $R^6$, m und n die oben angegebene Bedeutung haben und

Y für Halogen, sowie die Gruppierung $-O-SO_2-Z$ steht, wobei

Z für Methyl oder p-Methylphenyl steht,

mit Alkylisalzen von Azolen der Formel

$$M - Az \qquad (III)$$

in welcher

Az die oben angegebene Bedeutung hat und

M für ein Alkalimetall steht,

in Gegenwart eines Verdünnungsmittels umsetzt, oder

b) Azolylmethyl-keto-Derivate der Formel

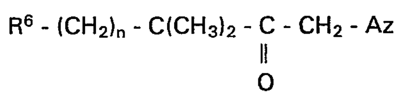
(IV)

in welcher

Az, $R^6$ und n die oben angegebene Bedeutung haben, mit Diolen der Formel

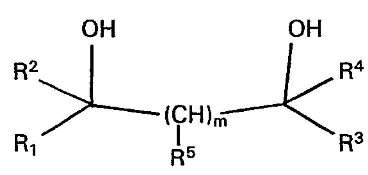
(V)

in welcher
$R^1$ bis $R^5$ und m die oben angegebene Bedeutung haben,
in Gegenwart eines Verdünnungsmittels und in Gegenwart einer Säure als Katalysator umsetzt, und gegebenenfalls noch an die nach Verfahrensvariante (a) oder (b) erhaltenen Verbindungen der Formel (I) anschliessend eine Säure oder ein Metallsalz addiert.

4. Fungizide Mittel, gekennzeichnet durch einen Gehalt an mindestens einem 2-Azolyl-methyl-1,3--dioxolan- und/oder -dioxan-Derivat der Formel (I) in Ansprüchen 1 und 3.

5. Verwendung von 2-Azolyl-methyl-1,3-dioxolan- oder -dioxan-Derivaten der Formel (I) in Ansprüchen 1 und 3 zur Bekämpfung von phytopathogenen Pilzen.

**Claims**

1. 2-Azolylmethyl-1,3-dioxolane and -dioxane derivatives of the general formula

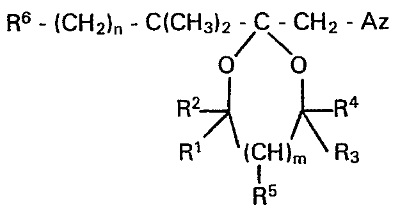

(I)

in which
Az represents imidazol-1-yl or 1,2,4-triazol-1-yl,
$R^1$ represents hydrogen or alkyl with 1 to 4 carbon atoms,
$R^2$ represents hydrogen or alkyl with 1 to 4 carbon atoms,
$R^3$ represents hydrogen or alkyl with 1 to 4 carbon atoms,
$R^4$ represents hydrogen or straight-chain or branched alkyl with 1 to 4 carbon atoms which can optionally be substituted by: hydroxyl, alkoxy with 1 to 4 carbon atoms, dialkylamino, dialkylaminocarbonyl or alkylcarbonyloxy with in each case 1 to 2 carbon atoms in each alkyl part, optionally substituted phenoxy or optionally substituted phenylalkoxy with 1 to 4 carbon atoms in the alkyl part, optionally substituted Phenylcarbonyloxy or optionally substituted phenylalkylcarbonyloxy with 1 to 4 carbon atoms in the alkyl part, alkylsulphonyloxy with 1 to 4 carbon atoms or optionally substituted phenylsulphonyloxy, the following being mentioned as the possible phenyl or phenoxy substituents in each case: halogen, alkyl with 1 to 4 carbon atoms, alkoxy and alkylthio with in each case 1 to 2 carbon atoms, halogenalkyl, halogenalkoxy and halogenoalkylthio with in each case 1 to 2 carbon atoms and 1 to 5 identical or different halogen atoms, such as, in particular, fluorine and chlorine atoms, dimethylamino, acetylamino, acetylmethylamino, and piperazinyl which is optionally substituted by methyl or acetyl; or finally represents optionally substituted phenyl or phenylalkyl with 1 to 4 carbon atoms in the alkyl part, possible phenyl substituents being the above-mentioned phenyl substituents;
$R^1$ and $R^3$ furthermore conjointly represent a tetramethylene or pentamethylene bridge which is optionally substituted by alkyl with 1 to 4 carbon atoms;
$R^5$ represents hydrogen or alkyl with 1 to 4 carbon atoms;
m represents the number 0 or 1;
$R^6$ represents hydrogen, halogen, cyano, straight-chain or branched alkyl with 1 to 4 carbon atoms or optionally substituted phenyl, possible phenyl substituents being the phenyl substituents already mentioned under $R^4$, or represents the grouping $-X-R^7$, $-COOR^8$ or $-CONHR^9$, wherein
X represents oxygen, sulphur, the SO group or the $SO_2$ group;
$R^7$ represents straight-chain or branched alkyl with 1 to 4 carbon atoms, halogenoalkyl with 1 to 2 carbon atoms and 1 to 5 identical or different halogen atoms, cyano, or optionally substituted phenyl or phenylalkyl with 1 to 4 carbon atoms in the alkyl part, possible phenyl substituents in each case being the phenyl substituents already mentioned und $R^4$;
$R^8$ represents straight-chain or branched alkyl with 1 to 4 carbon atoms;
$R^9$ represents straight-chain or branched alkyl with 1 to 4 carbon atoms or optionally substituted phenyl, possible phenyl substituents being the phenyl substituents already mentioned under $R^4$, and
n represents the number 0 or 1,
with the proviso, however, that the radical $R^6$-$-(CH_2)_n-$ may not represent methyl if m represents the number 0, Az represents the 1,2,4-triazol-1-yl radical and $R^4$ represents optionally substituted phenoxyalkyl,
as well as their plant-tolerated acid addition salts and metal salt complexes.

2. Compounds of the general formula (I) in claim 1, in which
$R^1$ represents hydrogen or straight-chain or branched alkyl with 1 to 4 carbon atoms;
$R^2$ represents hydrogen or straight-chain or branched alkyl with 1 to 4 carbon atoms;
$R^3$ represents hydrogen or straight-chain or branched alkyl with 1 to 4 carbon atoms,
$R^4$ represents hydrogen or straight-chain or branched alkyl with 1 to 4 carbon atoms which can optionally be substituted by: hydroxyl, methoxy, ethoxy, dimethylamino, dimethylaminocarbonyl, optionally substituted phenoxy or benzyloxy, methylcarbonyloxy, ethylcarbonyloxy, optionally substituted phenylcarbonyloxy or benzylcarbonyloxy, methylsulphonyloxy, ethylsulphonyloxy or optionally substituted phenylsulphonyloxy, the following being mentioned as the possible phenyl or phenoxy substituents in each case: fluorine, chlorine, methyl, ethyl, methoxy, methylthio, trifluoromethyl, trifluoromethylthio, dimethylamino, acetylamino, acetylmethylamino and 4-acetyl-piperazin-1-yl, or represents optionally substituted phenyl or benzyl, possible phenyl substituents being those mentioned above;

$R^1$ and $R^3$ furthermore conjointly represent a tetramethylene bridge,

$R^5$ represents hydrogen, methyl or ethyl;

$R^6$ represents hydrogen, fluorine, chlorine, bromine, methyl, ethyl, propyl, optionally substituted phenyl, possible substituents being the phenyl substituents already mentioned under $R^4$, or represents the grouping $-X-R^7$, $-COOR^8$ or $-CONHR^9$, wherein

$R^7$ represents methyl, ethyl, trifluoromethyl, cyano, or optionally substituted phenyl or benzyl, possible phenyl substituents in each case being the phenyl substituents mentioned under $R^4$;

$R^8$ represents methyl or ethyl;

$R^9$ represents methyl, ethyl or optionally substituted phenyl, possible phenyl substituents being the phenyl substituents already mentioned under $R^4$; and

n represents the number 1; and

Az, X and m have the meaning indicated in claim 1; with the proviso, however, that the radical $R^6$-$-(CH_2)_n-$ may not represent methyl if m represents the number 0, Az represents the 1,2,4-triazol-1-yl radical and $R^4$ represents optionally substituted phenoxyalkyl.

3. Process for the preparation of 2-azolylmethyl-1,3-dioxolane and -dioxane derivatives of the general formula

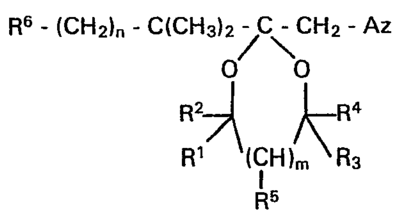

(I)

in which

Az represents imidazol-1-yl or 1,2,4-triazol-1-yl,

$R^1$ represents hydrogen or alkyl with 1 to 4 carbon atoms,

$R^2$ represents hydrogen or alkyl with 1 to 4 carbon atoms,

$R^3$ represents hydrogen or alkyl with 1 to 4 carbon atoms,

$R^4$ represents hydrogen or straight-chain or branched alkyl with 1 to 4 carbon atoms which can optionally be substituted by: hydroxyl, alkoxy with 1 to 4 carbon atoms, dialkylamino, dialkylaminocarbonyl or alkylcarbonyloxy with in each case 1 to 2 carbon atoms in each alkyl part, optionally substituted phenoxy or optionally substituted phenylalkoxy with 1 to 4 carbon atoms in the alkyl part, optionally substituted Phenylcarbonyloxy or optionally substituted phenylalkylcarbonyloxy with 1 to 4 carbon atoms in the alkyl part, alkylsulphonyloxy with 1 to 4 carbon atoms or optionally substituted phenylsulphonyloxy, the following being mentioned as the possible phenyl or phenoxy substituents in each case: halogen, alkyl with 1 to 4 carbon atoms, alkoxy and alkylthio with in each case 1 to 2 carbon atoms, halogenalkyl, halogenalkoxy and halogenoalkylthio with in each case 1 to 2 carbon atoms and 1 to 5 identical or different halogen atoms, such as, in particular, fluorine and chlorine atoms, dimethylamino, acetylamino, acetylmethylamino, and piperazinyl which is optionally substituted by methyl or acetyl; or finally represents optionally substituted phenyl or phenylalkyl with 1 to 4 carbon atoms in the alkyl part, possible phenyl substituents being the abovementioned phenyl substituents;

$R^1$ and $R^3$ furthermore conjointly represent a tetramethylene or pentamethylene bridge which is optionally substituted by alkyl with 1 to 4 carbon atoms;

$R^5$ represents hydrogen or alkyl with 1 to 4 carbon atoms;

m represents the number 0 or 1;

$R^6$ represents hydrogen, halogen, cyano, straight-chain or branched alkyl with 1 to 4 carbon atoms or optionally substituted phenyl, possible phenyl substituents being the phenyl substituents already mentioned under $R^4$, or represents the grouping $-X-R^7$, $-COOR^8$ or $-CONHR^9$, wherein

X represents oxygen, sulphur, the SO group or the $SO_2$ group;

$R^7$ represents straight-chain or branched alkyl with 1 to 4 carbon atoms, halogenoalkyl with 1 to 2 carbon atoms and 1 to 5 identical or different halogen atoms, cyano, or optionally substituted phenyl or phenylalkyl with 1 to 4 carbon atoms in the alkyl part, possible phenyl substituents in each case being the phenyl substituents already mentioned und $R^4$;

$R^8$ represents straight-chain or branched alkyl with 1 to 4 carbon atoms;

$R^9$ represents straight-chain or branched alkyl with 1 to 4 carbon atoms or optionally substituted phenyl, possible phenyl substituents being the phenyl substituents already mentioned under $R^4$, and

n represents the number 0 or 1,

with the proviso, however, that the radical $R^6$-$-(CH_2)_n-$ may not represent methyl if m represents the number 0, Az represents the 1,2,4-triazol-1-yl radical and $R^4$ represents optionally substituted phenoxyalkyl,

as well as their plant-tolerated acid addition salts and metal salt complexes, characterized in that

a) substituted 1,3-dioxolane and dioxane derivatives of the formula

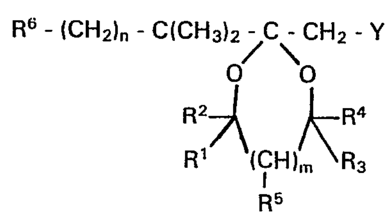

(II)

in which

$R^1$ to $R^6$, m and n have the abovementioned meaning and

Y represents halogen, or the grouping $-O-SO_2-Z$, wherein

Z represents methyl or p-methylphenyl,

are reacted with alkali metal salts of azoles of the formula

M - Az (III)

in which
Az has the abovementioned meaning and
M represents an alkali metal,
in the presence of a diluent, or
b) azolylmethyl-keto derivatives of the formula

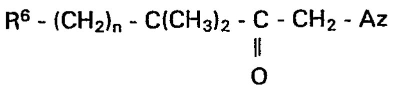 (IV)

in which
Az, $R^6$ and n have the abovementioned meaning,
are reacted with diols of the formula

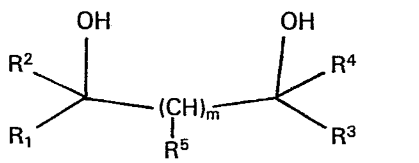 (V)

in which
$R^1$ to $R^5$ and m have the abovementioned meaning, in the presence of a diluent and in the presence of an acid as the catalyst, and, if required, an acid or metal salt is then added on to the compound of the formula (I) obtained according to process variant (a) or (b).

4. Fungicidal agents, characterised in that they contain at least one 2-azolyl-methyl-1,3-dioxolane and/or -dioxane derivative of the formula (I) in claims 1 and 3.

5. Use of 2-azolyl-methyl-1,3-dioxolane or -dioxane derivatives of the formula (I) in claims 1 and 3 for combating phytopathogenic fungi.

**Revendications**

1. Dérivés de 2-azolylméthyl-1,3-dioxolanne et -dioxanne de formule générale

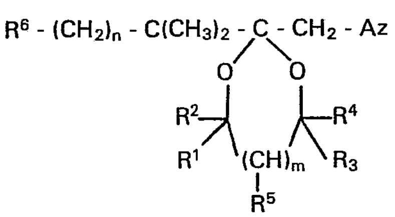 (I)

dans laquelle
Az représente un groupe imidazol-1-yle ou 1,2,4-triazol-1-yle,
$R^1$ représente un atome d'hydrogène ou un groupe alkyle ayant 1 à 4 atomes de carbone,
$R^2$ représente un atome d'hydrogène ou un groupe alkyle ayant 1 à 4 atomes de carbone,
$R^3$ représente un atome d'hydrogène ou un groupe alkyle ayant 1 à 4 atomes de carbone,
$R^4$ représente un atome d'hydrogène ou un groupe alkyle linéaire ou ramifié ayant 1 à 4 atomes de carbone (qui peut éventuellement être substitué par un groupe hydroxy, alcoxy ayant 1 à 4 atomes de carbone, dialkylamino, dialkylaminocarbonyle et alkylcarbonyloxy ayant chacun 1 à 2 atomes de carbone dans chaque partie alkyle, phénoxy éventuellement substitué et phénylalcoxy éventuellement substitué comportant 1 à 4 atomes de carbone dans la partie alkyle, phénylcarbonyloxy éventuellement substitué et phénylalkylcarbonyloxy éventuellement substitué et comportant 1 à 4 atomes de carbone dans la partie alkyle, alkylsulfonyloxy ayant 1 à 4 atomes de carbone et phénylsulfonyloxy éventuellement substitué, (et l'on peut citer pour les noyaux phényle ou phénoxy, comme substituants, un halogène, un groupe alkyle ayant 1 à 4 atomes de carbone, alcoxy et alkylthio ayant chacun 1 à 2 atomes de carbone, halogénoalkyle, halogénoalcoxy et halogénoalkylthio ayant chacun 1 à 2 atomes de carbone et 1 à 5 atomes d'halogène identiques ou différents, comme en particulier des atomes de fluor et de chlore, un groupe diméthylamino, acétylamino, acétylméthylamino, pipérazinyle éventuellement substitué par un groupe méthyle ou acétyle), et enfin un groupe phényle et phénylalkyle éventuellement substitué et comportant 1 à 4 atomes de carbone dans la partie alkyle, et l'on peut citer comme substituant du noyau phényle les substituants précités du noyau phényle;
$R^1$ et $R^3$ représentent en outre, quand ils sont pris ensemble, un pont tétra- ou penta-méthylène éventuellement substitué par un groupe alkyle ayant 1 à 4 atomes de carbone;
$R^5$ représente un atome d'hydrogène ou un groupe alkyle ayant 1 à 4 atomes de carbone;
m représente des nombres 0 ou 1;
$R^6$ représente un atome d'hydrogène ou d'halogène ou un groupe cyano, alkyle linéaire ou ramifié comportant 1 à 4 atomes de carbone et phényle éventuellement substitué et l'on peut citer comme substituants du noyau phényle les substituants du noyau phényle déjà cités pour $R^4$, et $R^6$ représente également les groupements $-X-R^7$, $-COOR^8$ et $-CONHR^9$, où
X représente un atome d'oxygène ou de soufre ou le groupe SO- ou $SO_2$-;
$R^7$ représente un groupe alkyle linéaire ou ramifié ayant 1 à 4 atomes de carbone, halogénoalkyle ayant 1 à 2 atomes de carbone et 1 à 5 atomes d'halogène identiques ou différents, un groupe cyano, un groupe phényle et phénylalkyle éventuellement substitué comportant 1 à 4 atomes de carbone dans la partie alkyle; et l'on peut citer chaque fois comme substituants du noyau phényle les substituants du noyau phényle déjà cités pour $R^4$;
$R^8$ représente un groupe alkyle linéaire ou ramifié ayant 1 à 4 atomes de carbone;
$R^9$ représente un groupe alkyle linéaire ou ramifié ayant 1 à 4 atomes de carbone ainsi qu'un groupe phényle éventuellement substitué et l'on peut citer comme substituants du noyau phényle les substituants du noyau phényle déjà cités pour $R^4$; et

n représente les nombres 0 ou 1;

étant bien entendu que le radical $R^6$-$(CH_2)_n$- ne doit pas représenter un groupe méthyle lorsque m est nul, Az représente le radical 1,2,4-triazol-1-yle et $R^4$ un groupe phénoxyalkyle éventuellement substitué, ainsi que leurs sels d'addition d'acides et complexes des sels de métaux tolérables par les plantes.

2. Composés selon la formule générale (I) de la revendication 1,

dans laquelle

$R^1$ représente un atome d'hydrogène ou un groupe alkyle linéaire ou ramifié ayant 1 à 4 atomes de carbone;

$R^2$ représente un atome d'hydrogène ou un groupe alkyle linéaire ou ramifié ayant 1 à 4 atomes de carbone;

$R^3$ représente un atome d'hydrogène ou un groupe alkyle linéaire ou ramifié ayant 1 à 4 atomes de carbone;

$R^4$ représente un atome d'hydrogène ou un groupe alkyle linéaire ou ramifié ayant 1 à 4 atomes de carbone (qui peut éventuellement être substitué parun groupe hydroxyle, méthoxy, éthoxy, diméthylamino, diméthylaminocarbonyle, phénoxy et benzyloxy éventuellement substitué, méthylcarbonyloxy, éthylcarbonyloxy, phénylcarbonyloxy et benzylcabonyloxy éventuellement substitué, méthylsulfonyloxy, éthylsulfonyloxy ainsi que phénylsulfonyloxy éventuellement substitué et l'on peut citer chaque fois comme substituants du noyau phényle ou phénoxy: un atome de fluor ou de chlore ou un groupe méthyle, éthyle, méthoxy, méthylthio, trifluorométhyle, trifluorométhylthio, diméthylamino, acétylamino, acétylméthylamino et 4-acétylpipérazine-1-yle, ainsi qu'un groupe phényle et benzyle éventuellement substitué, les substituants du noyau phényle étant ceux indiqués ci-dessus;

$R^1$ et $R^3$ peuvent en outre aussi, lorsqu'ils sont pris ensemble, représenter un pont tétraméthylène;

$R^5$ représente un atome d'hydrogène ou un groupe méthyle ou éthyle;

$R^6$ représente un atome d'hydrogène, de fluor, de chlor ou de brome ou un groupe méthyle, éthyle, propyle, phényle éventuellement substitué (et l'on peut citer comme substituants les substituants du noyau phényle déjà cités pour $R^4$), ainsi que les groupements -X-$R^7$, -COO$R^8$ et -CONH$R^9$, où

$R^7$ représente un groupe méthyle, éthyle, trifluorométhyle, cyano, ainsi que phényle et benzyle éventuellement substitué, et l'on peut citer chaque fois comme substituants du noyau phényle les substituants du noyau phényle déjà cités pour $R^4$;

$R^8$ représente un groupe méthyle ou éthyle;

$R^9$ représente un groupe méthyle, éthyle, ainsi que phényle éventuellement substitué et l'on peut citer comme substituants du noyau phényle les substituants du noyau phényle déjà cités pour $R^4$; et

n représente les nombre1; et

Az, X et m ont le sens indiqué à la revendication 1; mais, étant bien entendu, que le radical $R^6$-$(CH_2)_n$- ne doit pas représenter un groupe méthyle lorsque m est nul, Az représente le radical 1,2,4-triazol-1-yle et $R^4$ un groupe phénoxyalkyle éventuellement substitué.

3. Procédé de préparation de dérivés de 2-azolylméthyl-1,3-dioxolannes et -dioxannes, de formule générale

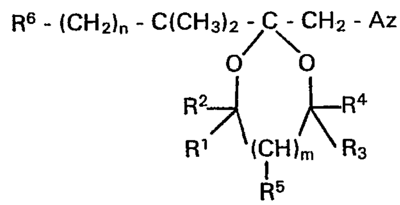

(I)

dans laquelle

Az représente un groupe imidazol-1-yle ou 1,2,4-triazol-1-yle,

$R^1$ représente un atome d'hydrogène ou un groupe alkyle ayant 1 à 4 atomes de carbone,

$R^2$ représente un atome d'hydrogène ou un groupe alkyle ayant 1 à 4 atomes de carbone,

$R^3$ représente un atome d'hydrogène ou un groupe alkyle ayant 1 à 4 atomes de carbone,

$R^4$ représente un atome d'hydrogène ou un groupe alkyle linéaire ou ramifié ayant 1 à 4 atomes de carbone (qui peuvent éventuellement être substitué par un groupe hydroxyle, alcoxy ayant 1 à 4 atomes de carbone, dialkylamino, dialkylaminocarbonyle et alkylcarbonyloxy ayant chacun 1 à 2 atomes de carbone dans chaque partie alkyle, phénoxy éventuellement substitué et phénylalcoxy éventuellement substitué comportant 1 à 4 atomes de carbone dans la partie alkyle, phénylcarbonyloxy éventuellement substitué et phénylalkylcarbonyloxy éventuellement substitué et comportant 1 à 4 atomes de carbone dans la partie alkyle, alkylsulfonyloxy ayant 1 à 4 atomes de carbone et phénylsulfonyloxy éventuellement substitué, et l'on peut citer comme substituants du noyaux phényle ou phénoxy, un atome d'halogène, un groupe alkyle ayant 1 à 4 atomes de carbone, alcoxy et alkylthio ayant chacun 1 à 2 atomes de carbone, halogénoalkyle, halogénoalcoxy et halogénoalkylthio ayant chacun 1 à 2 atomes de carbone et 1 à 5 atomes d'halogène identiques ou différents, comme en particulier des atomes de fluor et de chlore, un groupe diméthylamino, acétylamino, acétylméthylamino, pipérazinyle éventuellement substitué par un groupe méthyle ou acétyle, et enfin un groupe phényle et phénylalkyle éventuellement substitué et comportant 1 à 4 atomes de carbone dans la partie alkyle, et l'on peut citer substituant du noyau phényle les substituants précités du noyau phényle;

$R^1$ et $R^3$ peuvent en outre, quand ils sont pris ensemble, représenter un pont tétra- ou penta-méthylène éventuellement substitué par un groupe alkyle ayant 1 à 4 atomes de carbone;

$R^5$ représente un atome d'hydrogène ou un groupe alkyle ayant 1 à 4 atomes de carbone;

m représente des nombres 0 ou 1;

$R^6$ représente un atome d'hydrogène ou d'halogène ou un groupe cyano, alkyle linéaire ou ramifié

comportant 1 à 4 atomes de carbone et phényle éventuellement substitué et l'on peut citer comme substituants du noyau phényle les substituants du noyau phényle déjà cités pour $R^4$, ainsi que les groupes -X-$R^7$, -$COOR^8$ et -$CONHR^9$, où

X représente un atome d'oxygène ou de soufre ou le groupe SO- ou $SO_2$-;

$R^7$ représente un groupe alkyle linéaire ou ramifié ayant 1 à 4 atomes de carbone, halogénoalkyle ayant 1 à 2 atomes de carbone et 1 à 5 atomes d'halogène identiques ou différents, un groupe cyano, ainsi qu'un groupe phényle et phénylalkyle éventuellement substitué et comportant 1 à 4 atomes de carbone dans la partie alkyle; et l'on peut citer chaque fois comme substituants du noyau phényle les substituants du noyau phényle déjà cités pour $R^4$;

$R^8$ représente un groupe alkyle linéaire ou ramifié ayant 1 à 4 atomes de carbone;

$R^9$ représente un groupe alkyle linéaire ou ramifié ayant 1 à 4 atomes de carbone ainsi qu'un groupe phényle éventuellement substitué et l'on peut citer comme substituants du noyau phényle les substituants du noyau phényle déjà cités pour $R^4$; et

$\underline{n}$ représente les nombres 0 ou 1;

étant bien entendu que le radical $R^6$-$(CH_2)_n$- ne doit pas représenter un groupe méthyle lorsque $\underline{m}$ est nul, Az représente le radical 1,2,4-triazol-1-yle et $R^4$ un groupe phénoxyalkyle éventuellement substitué, ainsi que leurs sels d'addition d'acides et de complexes de sels de métaux tolérables par les plantes, procédé caractérisé en ce que

a) on fait réagir des dérivés de 1,3-dioxolannes- et de dioxannes substitués, de formule

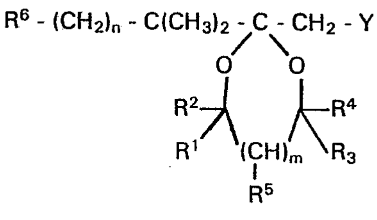

(II)

dans laquelle

$R^1$ à $R^6$, $\underline{m}$ et $\underline{n}$ ont le sens indiqué ci-dessus et

Y représente un atome d'halogène, ainsi qu'un groupement -O-$SO_2$-Z, dans lequel Z représente un groupe méthyle ou p-méthylphényle avec des sels alcalins d'azoles de formule

$$M - Az \qquad (III)$$

dans laquelle

Az a le sens précité et

M représente un métal alcalin,

en présence d'un diluant, ou

b) on fait réagir des dérivés azolylméthylcétoniques de formule

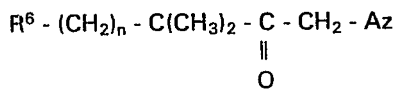

(IV)

dans laquelle

Az, $R^6$ et $\underline{n}$ ont le sens précité,

avec des diols de formule

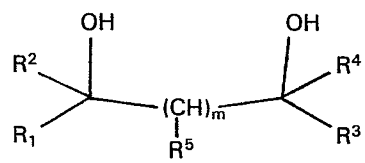

(V)

dans laquelle

$R^1$ à $R^5$ et $\underline{m}$ ont le sens précité,

en présence d'un diluant et en présence d'un acide comme catalyseur et l'on fixe éventuellement encore ensuite, par addition, un acide ou un sel de métal sur les composés de formule (I) obtenus selon la variante (a) ou (b) de procédé.

4. Produit fongicide, caractérisé en ce qu'il contient au moins un dérivé de 2-azolylméthyl-1,3-dioxolanne et/ou -dioxanne de formule (I) des revendications 1 et 3.

5. Utilisation des dérivés de 2-azolylméthyl-1,3--dioxolannes ou -dioxannes de formule (I) des revendications 1 et 3 pour la lutte contre les champignons phytopathogènes.